# EUROPEAN PATENT SPECIFICATION

(11) **EP 3 988 038 B1**
(45) Date of publication and mention of the grant of the patent: **18.09.2024**
(21) Application number: 21202112.5
(22) Date of filing: 12.10.2021
(51) Int. Cl.: A61B 17/16

(54) **PRESSURE LOADED DRIVE CONTROL FOR BONE RESECTION**
DRUCKBELASTETE ANTRIEBSREGELUNG FÜR KNOCHENRESEKTION
COMMANDE D'ENTRAÎNEMENT SOUS PRESSION POUR LA RÉSECTION OSSEUSE

(30) Priority: 22.10.2020 US 202017077603
(43) Date of publication of application: 27.04.2022
(73) Proprietor: Medtronic Xomed, Inc., Jacksonville, FL 32216 (US)
(72) Inventor: VU, Michael, Jacksonville, 32216 (US); BARNES, Milton F., Jacksonville, 32216 (US); HARIHARESAN, Seralaathan, Jacksonville, 32216 (US); MALLA, Aayush, Jacksonville, 32216 (US)
(74) Representative: Viering, Jentschura & Partner mbB Patent- und Rechtsanwälte

(56) References cited:
- EP-A1- 3 287 083
- CN-A- 104 323 816
- DE-U1- 7 911 715
- GB-A- 2 086 279
- US-A- 4 951 690
- US-A1- 2019 142 439

## Description

### FIELD

The present disclosure relates to a drill assembly and to a method for cutting an object.

### BACKGROUND

This section provides background information related to the present disclosure, which is not necessarily prior art.

It is important to take precautions when cutting around sensitive anatomy. While existing cutting devices, such as bone drills, are suitable for their intended use, they are subject to improvement. The present disclosure advantageously includes a cutting device with a safety mechanism for use in anatomical and non-anatomical applications. One skilled in the art will appreciate that the present disclosure includes numerous additional advantages and unexpected results as well.

US 4 951 690 A discloses a drill-type cranial perforator of the type which comprises a front drill head assembly made up of a leading inner drill and a trailing outer drill, and a rear support and drive assembly adapted to enable both drills so long as the leading inner drill is encountering a resistive surface and to disable both drills when the leading inner drill stops encountering the resistive surface. The leading inner drill has at least one drilling flute with a forward cutting edge including a reentrant cutting segment axially rearward from the outer end of the forward cutting edge and extending inwardly towards the longitudinal axis of the perforator.

### SUMMARY

The invention provides a drill assembly according to claim 1 and a method for cutting a non anatomical object according to claim 7. Further embodiments are described in the dependent claims. Methods for cutting anatomical objects are also described herein, but they do not form part of the invention as claimed.

### DRAWINGS

The drawings described herein are for illustrative purposes only of select embodiments and not all possible implementations, and are not intended to limit the scope of the present disclosure.
FIG.1 illustrates exemplary use of a drill assembly attachment in accordance with the present disclosure;
FIG. 2 is a perspective view of the attachment of FIG. 1;
FIG. 3A is a cross-sectional view of the attachment in an active configuration for cutting an object;
FIG. 3B illustrates a pressure loaded drive control assembly of the attachment in the active configuration in additional detail;
FIG. 4A illustrates the attachment in an inactive configuration subsequent to cutting through the object;
FIG. 4B illustrates the pressure loaded drive control assembly of the attachment in the inactive configuration in additional detail;
FIG. 5A illustrates another drill assembly attachment in accordance with the present disclosure;
FIG. 5B illustrates the attachment of FIG. 5A in an active configuration to cut the object;
FIG. 5C illustrates the attachment of FIG. 5A in an inactive configuration subsequent to cutting through the object;
FIG. 6 is a perspective view of another pressure loaded drive control assembly in accordance with the present disclosure;
FIG. 7 illustrates the pressure loaded drive control assembly of the present disclosure within a housing for insertion into a drill attachment; and
FIG. 8 illustrates another drill assembly attachment in accordance with the present disclosure including a cutting tool in the form of a saw.

Corresponding reference numerals indicate corresponding parts throughout the several views of the drawings.

### DETAILED DESCRIPTION

Example embodiments will now be described more fully with reference to the accompanying drawings.

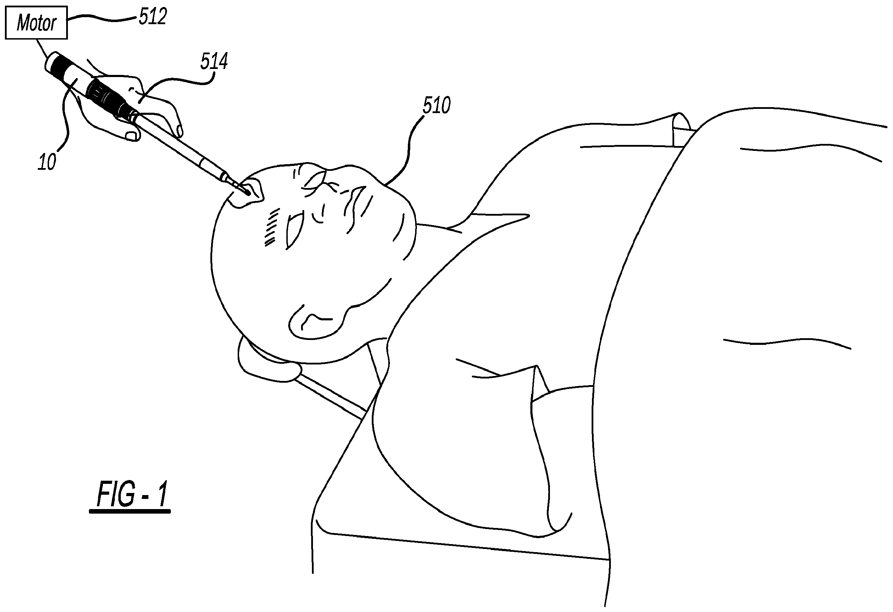

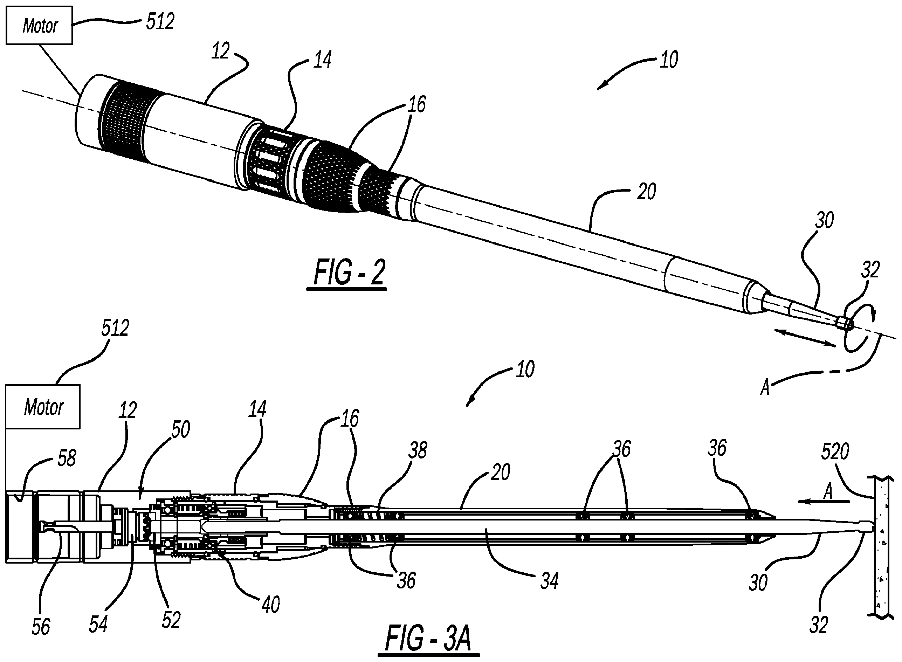

With initial reference to FIGS. 1 and 2, an exemplary drill assembly attachment in accordance with the present disclosure is illustrated at reference numeral 10. The attachment 10 is configured for use to cut any suitable anatomical or non-anatomical objects. As illustrated in the example of FIG. 1, the attachment 10 may be used to cut bone of a patient 510, such as the patient's cranium. The attachment 10 is connected to any suitable motor 512, and is directed to the patient 510 by a user 514, such as a surgeon, or any automated surgical navigation device. In non-anatomical applications, the user 514 may be any person or automated device capable of using the attachment 10 to cut an object.

With particular reference to FIG. 2, the attachment 10 generally includes a base 12, which is configured to be connected to the motor 512. A locking collar 14 is adjacent to the base 12. Rotation of the locking collar 14 rotationally locks a cutting tool 30 to the attachment 10. The motor 512 may be any suitable pneumatic motor, electric motor, etc.

Proximate to the locking collar 14 is a grip collar 16, which provides a roughened surface to facilitate grip of the attachment 10 by the user 514. Extending from the grip collar 16 is a tube 20 into which the cutting tool 30 is inserted. The cutting tool 30 will typically include a cutting head 32. The cutting tool 30 may be any suitable cutting tool. Suitable cutting tools include any suitable drill bits, burs, and saw, for example. The cutting tool 30 may be configured for any suitable plunge cut or lateral cut. With respect to plunge cuts and as described in detail herein, the cutting tool 30 is slidably or axially movable along a longitudinal axis A of the attachment 10. In some applications, the cutting tool 30 is also rotatable about the longitudinal axis A.

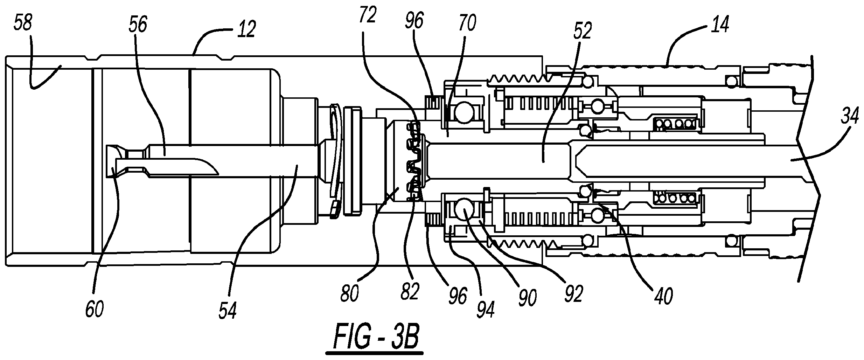

With additional reference to FIGS. 3A and 3B, additional details of the attachment 10 will now be described. The attachment 10 further includes a tool shaft 34 defined by the tube 20. The tool 30 extends through the tool shaft 34, through the grip collar 16, and into the locking collar 14. The tool shaft 34 is supported within the tube 20 by one or more bearings 36. The bearings 36 closest to the grip collar 16 are preloaded by a spring 38 to ensure that the bearings 36 do not overheat during use. Within the locking collar 14 is a locking assembly 40. The locking assembly 40 is any suitable locking mechanism configured to lock the tool 30 to the attachment 10, and allow the tool 30 to be unlocked and replaced with another tool.

The attachment 10 further includes a pressure loaded drive control assembly 50. The pressure loaded drive control assembly 50 generally includes a driven member 52 and a drive member 54. The driven member 52 is slidably mounted within the attachment 10, and axially slides in unison with the tool 30 along the longitudinal axis A. The drive member 54 is configured to be driven by the motor 512. The drive member 54 includes a drive shaft 56 accessible at a receptacle 58 defined by the base 12. A connector of the motor 512 is plugged into the receptacle 58 and placed into cooperation with a connector 60 of the drive shaft 56. Energy or force, generated by the motor 512 is transferred to the drive member 54 by way of the drive shaft 56 thereof. For example, rotational energy from the motor 512 is transferred to the driveshaft 56 for rotating the drive member 54.

FIG. 3B illustrates the pressure loaded drive control assembly 50 with additional detail. The pressure loaded drive control assembly 50 further includes a driven gear head 70 of the driven member 52, which has driven gear teeth 72. The drive member 54 further includes a drive gear head 80 having drive gear teeth 82. In the active configuration of FIG. 3B, the driven gear teeth 72 mesh with the drive gear teeth 82. Thus, in the exemplary active configuration of FIG. 3B, the drive member 54 rotates the driven member 52, which rotates the tool 30.

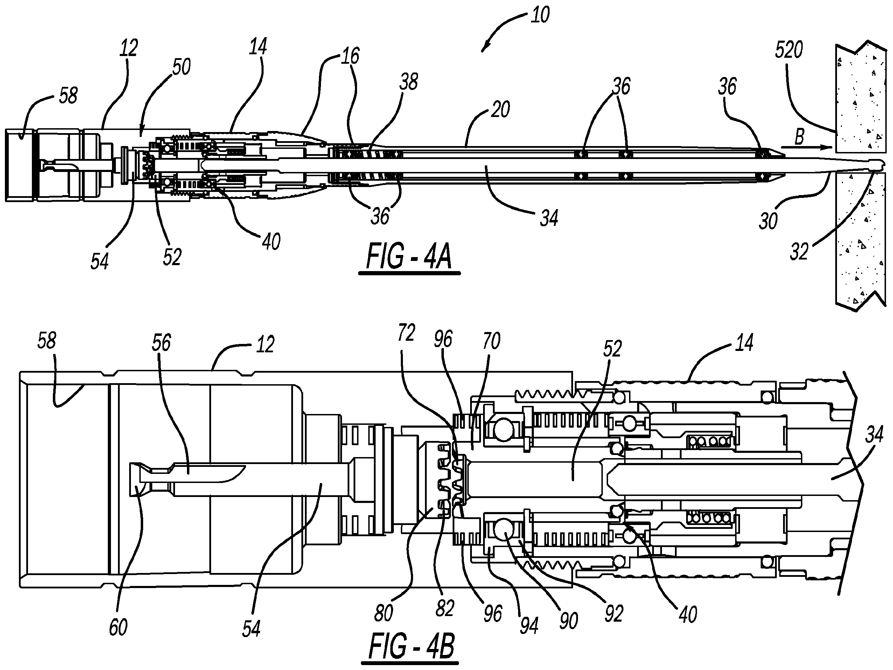

The pressure loaded drive control assembly 50 further includes a bearing 90 seated on a bearing case 92. The bearing case 92 is axially slidable along the longitudinal axis A. The bearing case 92 includes a bearing flange 94. A biasing member 96, such as a spring, is in direct or indirect cooperation with the bearing case 92, such as at the bearing flange 94. The biasing member 96 biases the bearing case 92 in an inactive configuration, which is described below in conjunction with the description of FIGS. 4A and 4B. The driven member 52 is connected to the bearing case 92, and axially moves along the longitudinal axis A with the bearing case 92. As a result, the driven member 52 is biased in the inactive configuration of FIGS. 4A and 4B by the biasing member 96. The driven member 52 also slides in unison with the tool 30 along the longitudinal axis A. The driven member 52 is connected directly to, or indirectly to, the tool 30.

FIG. 3A and 3B illustrate the attachment 10 in use with the head 32 of the tool 30 moved in a first direction A as a result of the head 32 being pressed or loaded against the object 520 to be cut. Depressing the attachment 10 against the object 520 applies pressure to the tool 30 to axially move the tool 30 along the longitudinal axis A further into the tube 20 in the first direction A. Because the driven member 52 moves with the tool 30, the driven member 52 also axially moves along the longitudinal axis A in the first direction A. The driven member 52 moves into cooperation with the drive member 54 such that the driven gear teeth 72 mesh with the drive gear teeth 92 for transferring energy or a force from the motor 512 to the tool 30 to drive the tool 30 for cutting the object 520, such as a rotational force.

FIGS. 4A and 4B illustrate the attachment 10 in the inactive configuration. The biasing member 96 moves the attachment 10 to the inactive configuration after the head 32 disengages or penetrates the object 520. In the inactive configuration, the tool 30 is no longer driven by the motor and no longer rotates, thus decreasing any possibility that objects and/or tissue beyond the object 520 will be cut by the head 32. Specifically, once the head 32 passes through the object 520, sufficient opposing axial pressure or force is no longer applied to the head 32. As soon as pressure or opposing axial force is not applied to the head 32, the biasing member 96 pushes the driven member 52 away from the drive member 54, which in turn axially moves the tool 30 along the longitudinal axis A in a second direction B such that the tool 30 extends further out from within the tube 20. In this inactive configuration, the driven gear teeth 72 are no longer in cooperation with the drive gear teeth 82, and thus energy or driven force is no longer transferred from the drive member 54 to the driven member 52. This idles the cutting tool 30 so that it is no longer rotated or driven by the motor, which advantageously provides an additional safety feature to further protect protects an area beyond the object 520 from being cut by the tool 30.

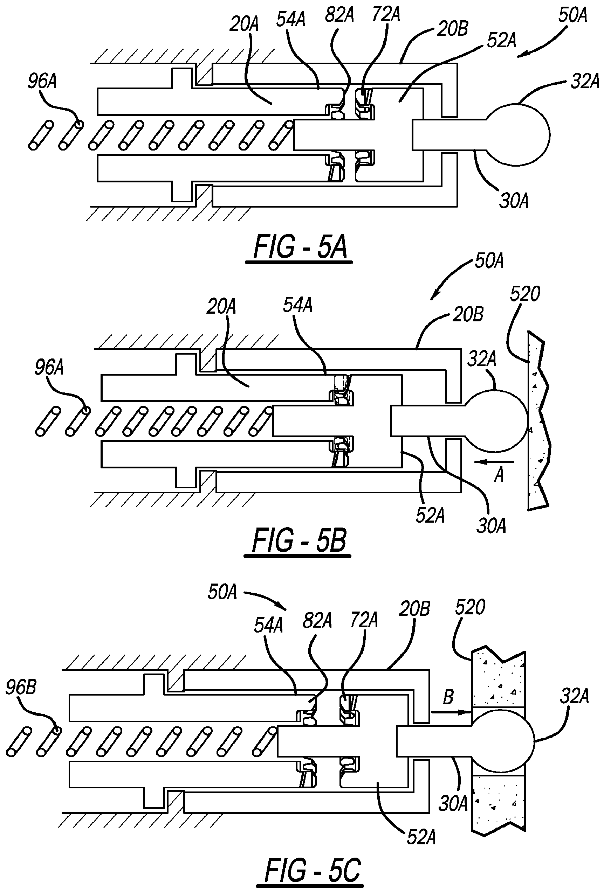

With reference to FIGS. 5A, 5B, and 5C, another pressure (i.e., force) loaded drive control assembly in accordance with the present disclosure is illustrated at reference numeral 50A. The drive control assembly 50A includes numerous features that are substantially similar to the drive control assembly 50, which are illustrated using the same reference numerals but also including the letter "A." The pressure loaded drive control assembly 50A is similar to the assembly 50, but the driven member 52A and the drive member 54A are both at a distal end of the tube 20. The driven member 52A is connected directly to the tool 30A, or is monolithic with the tool 30A. The biasing member 96A biases the driven member 52A in the inactive configuration such that the driven gear teeth 72A of the driven member 52A are spaced apart from (and decoupled from) the drive gear teeth 82A of the drive member 54A.

With reference to FIG. 5B, when the tool 30A is pressed against the object 520 to be cut, the driven member 52A is axially moved along the longitudinal axis A in direction A until the driven gear teeth 72A cooperate with the drive gear teeth 82A to rotate the tool 30A. With reference to FIG. 5C, after the head 32A cuts through the object 520, the head 32A is no longer under opposed axial loads or forces, which allows the biasing member 96A to decouple the driven and drive members 52A, 52B, and move the driven member 52A outward along the longitudinal axis A in direction B back to the inactive position. As a result, the driven gear teeth 72A are no longer in cooperation with the drive gear teeth 82A, the drive member 54A no longer drives the driven member 52A, and the tool 30 is not rotated.

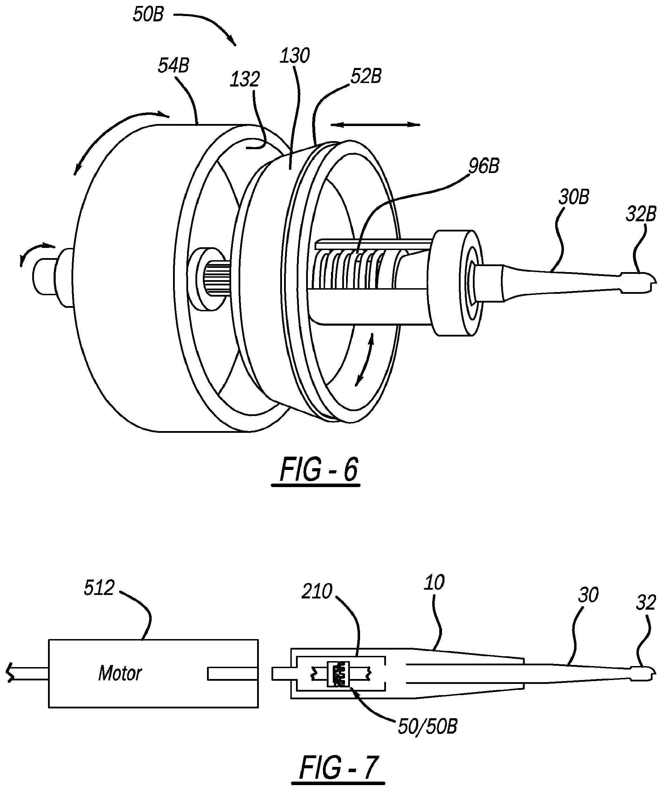

Although the driven members 52/52A and the drive members 54/54A are described above as including gear teeth, any other suitable coupling and decoupling configuration may be used to selectively transfer energy from the drive members 54/54A to the driven members 52/52A. For example and as illustrated in FIG. 6, the present disclosure further provides for a pressure loaded drive control assembly 50B. The assembly 50B is generally a friction clutch configuration. Specifically, the assembly 50B includes a driven member 52B having a driven surface 130, and a drive member 54B having a drive surface 132. In the inactive configuration of FIG. 6, the driven member 52B and the drive member 54B are spaced apart so that the tool 30B and the head 32B thereof are not rotated. The biasing member 96B biases the driven member 52B and the drive member 54B in this inactive, spaced apart configuration.

When the cutting tool 30B is depressed against the object 520 to be cut, the driven member 52B is moved along the longitudinal axis until the driven surface 130 is received within the drive surface 132. The driven surface 130 and the drive surface 132 each include any suitable surface treatments and/or inserts to provide a friction lock between the driven surface 130 and the drive surface 132. Thus, with the driven surface 130 seated within the drive surface 132, rotation of the drive member 54B by the motor 512 rotates driven member 52B and the cutting tool 30B. After the tool 30B cuts through the object 520, pressure is no longer applied against the cutting tool 30, which allows the biasing member 96 to move the driven member 52B back to the inactive configuration illustrated in FIG. 6 so that the cutting tool 30 is no longer driven.

With reference to FIG. 7, the pressure loaded drive control assembly 50, 50B may be self-contained within a housing 210. The housing 210 including the pressure loaded drive control assembly 50 or 50B may be removably coupled to any suitable existing drill assembly attachment 10' to "retrofit" the attachment 10 with a safety feature for reducing any possibility of unintentionally cutting sensitive tissue, organs, or non-anatomical material/device located beyond the object 520.

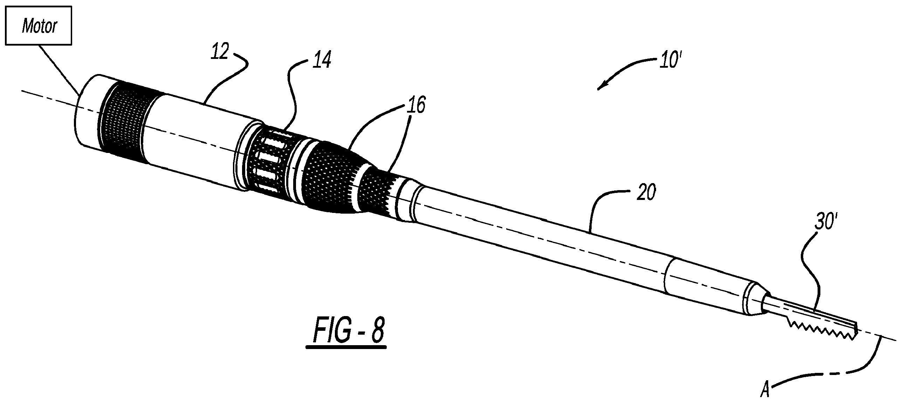

The pressure loaded drive control assemblies 50, 50A, 50B may be used with any suitable tool 30 in addition to a bur or drill tip. For example and as illustrated in FIG. 8, the tool 30 may include a saw tip 310. Any of the pressure loaded drive control assemblies 50, 50A, 50B may be used to control energy transfer to the saw tip 310 as described above and as one skilled in the art will appreciate.

The present disclosure thus advantageously provides for the drill assembly attachment 10 and the pressure loaded drive control assemblies 50, 50A, and 50B described above, which advantageously reduce any risk of cutting through the object 520 and damaging an anatomical organ, tissue, etc., or a non-anatomical object. With respect to anatomical applications, the present disclosure applies to use of the drill assembly attachment 10 to carry out any suitable procedure, such as the following examples: mastoidectomy, craniotomy, bur hole formation, pilot hole formation for spinal fusion, laminectomy/ laminectomies, bone resection, tissue resection, and robotic surgical procedures. The present disclosure provides an additional layer of safety and control when cutting around sensitive anatomy. With respect to robotic applications in particular, the present disclosure provides a primary layer of safety and control when cutting around sensitive areas instead of relying entirely on software control.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

## Claims

1. A drill assembly (10) comprising:
a cutting tool (30B) slidably movable along a longitudinal axis (A) of the drill assembly (10) and drivable to cut an object (520);
a pressure loaded drive control assembly (50B) configurable in an active configuration and an inactive configuration, in the active configuration the pressure loaded drive control assembly (50B) transfers energy from a motor (512) to the cutting tool (30B) to drive the cutting tool (30B), in the inactive configuration the pressure loaded drive control assembly (50B) prevents energy transfer from the motor (512) to the cutting tool (30B); and
a biasing member (96B) of the pressure loaded drive control assembly (50) configured to bias the pressure loaded drive control assembly (50) in the inactive configuration;
wherein depressing the cutting tool (30B) against the object (520) applies a load to the cutting tool (30B) to move the cutting tool (30B) along the longitudinal axis (A) in a first direction and moves the pressure loaded drive control assembly (50B) from the inactive configuration to the active configuration;
wherein the biasing member (96B) moves the cutting tool (30B) along the longitudinal axis (A) in a second direction that is opposite to the first direction and returns the pressure loaded drive control assembly (50B) to the inactive configuration from the active configuration when the load is no longer applied to the cutting tool (30B); and
**characterised in that** the pressure loaded drive control assembly (50B) is a friction clutch configuration.

2. The drill assembly (10) of Claim 1, wherein the cutting tool (30B) is configured to cut bone.

3. The drill assembly (10) of Claim 1 or 2, wherein the cutting tool (30B) is configured to cut a non-anatomical object.

4. The drill assembly (10) of one of Claims 1-3, wherein the cutting tool (30B) is one of a bur, a bit, and a saw.

5. The drill assembly (10) of one of Claims 1-4, wherein the pressure loaded drive control assembly (50B) includes a driven member (52B) movable into cooperation with the cutting tool (30B) to drive the cutting tool (30B), the driven member (52B) moves in unison with the cutting tool (30B) along the longitudinal axis (A) of the drill assembly (10).

6. The drill assembly (10) of one of Claims 1-5, wherein the biasing member (96B) includes a spring.

7. A method for cutting a non anatomical object (520), the method comprising:
connecting a drill assembly (10) according to any one of claims 1 to 6 to a motor (512) for driving a cutting tool (30B) of the drill assembly (10);
applying a load to the cutting tool (30B) by pressing the cutting tool (30B) against the object (520) to slidably move the cutting tool (30B) along a longitudinal axis (A) of the drill assembly (10) in a first direction, and to move a pressure loaded drive control assembly from an inactive configuration to an active configuration in which the pressure loaded drive control assembly (50B) transfers energy from the motor (512) to the cutting tool (30B) to drive the cutting tool (30B); and
cutting through the object (520) such that the cutting tool (30B) is relieved of the load, thereby allowing the biasing member (96B) to move the cutting tool (30B) along the longitudinal axis (A) in a second direction that is opposite to the first direction, and move the pressure loaded drive control assembly (50B) to the inactive configuration to not drive the cutting tool (30B).

8. The method of Claim 7, wherein in the active configuration a drive member (54B) is in cooperation with a driven member (52B) of the pressure loaded drive control assembly (50B).

9. The method of Claim 7 or 8, wherein in the inactive configuration the drive member (54B) is spaced apart from the driven member (52B) of the pressure loaded drive control assembly (50B).

10. The method of one of Claims 7-9, further comprising cutting through the object (520) with one of a bur, drill bit, and a saw.

## Patentansprüche

1. Bohranordnung (10), umfassend:
ein Schneidwerkzeug (30B), das entlang einer Längsachse (A) der Bohranordnung (10) verschiebbar bewegbar ist und antreibbar ist, um ein Objekt (520) zu schneiden;
eine druckbelastete Antriebssteueranordnung (50B), die in einer aktiven Konfiguration und einer inaktiven Konfiguration konfigurierbar ist, wobei in der aktiven Konfiguration die druckbelastete Antriebssteueranordnung (50B) Energie von einem Motor (512) auf das Schneidwerkzeug (30B) überträgt, um das Schneidwerkzeug (30B) anzutreiben, wobei in der inaktiven Konfiguration die druckbelastete Antriebssteueranordnung (50B) eine Energieübertragung von dem Motor (512) auf das Schneidwerkzeug (30B) verhindert; und
ein Vorspannelement (96B) der druckbelasteten Antriebssteueranordnung (50), das konfiguriert ist, um die druckbelastete Antriebssteueranordnung (50) in die inaktive Konfiguration vorzuspannen;
wobei Niederdrücken des Schneidwerkzeugs (30B) gegen das Objekt (520) eine Last auf das Schneidwerkzeug (30B) ausübt, um das Schneidwerkzeug (30B) entlang der Längsachse (A) in eine erste Richtung zu bewegen, und die druckbelastete Antriebssteueranordnung (50B) von der inaktiven Konfiguration in die aktive Konfiguration bewegt;
wobei das Vorspannelement (96B) das Schneidwerkzeug (30B) entlang der Längsachse (A) in eine zweite Richtung bewegt, die der ersten Richtung entgegengesetzt ist, und die druckbelastete Antriebssteueranordnung (50B) aus der aktiven Konfiguration in die inaktive Konfiguration zurückführt, wenn die Last nicht mehr auf das Schneidwerkzeug (30B) ausgeübt wird; und
**dadurch gekennzeichnet, dass** die druckbelastete Antriebssteueranordnung (50B) eine Reibungskupplungskonfiguration ist.

2. Bohranordnung (10) nach Anspruch 1, wobei das Schneidwerkzeug (30B) konfiguriert ist, um Knochen zu schneiden.

3. Bohranordnung (10) nach Anspruch 1 oder 2, wobei das Schneidwerkzeug (30B) konfiguriert ist, um ein nicht-anatomisches Objekt zu schneiden.

4. Bohranordnung (10) nach einem der Ansprüche 1 bis 3, wobei das Schneidwerkzeug (30B) eines von einer Fräse, einem Einsatz oder einer Säge ist.

5. Bohranordnung (10) nach einem der Ansprüche 1 bis 4, wobei die druckbelastete Antriebssteueranordnung (50B) ein angetriebenes Element (52B) einschließt, das in Zusammenwirkung mit dem Schneidwerkzeug (30B) bewegbar ist, um das Schneidwerkzeug (30B) anzutreiben, wobei sich das angetriebene Element (52B) gleichzeitig mit dem Schneidwerkzeug (30B) entlang der Längsachse (A) der Bohranordnung (10) bewegt.

6. Bohranordnung (10) nach einem der Ansprüche 1 bis 5, wobei das Vorspannelement (96B) eine Feder einschließt.

7. Verfahren zum Schneiden eines nicht-anatomischen Objekts (520), das Verfahren umfassend:
Verbinden einer Bohranordnung (10) nach einem der Ansprüche 1 bis 6 mit einem Motor (512) zum Antreiben eines Schneidwerkzeugs (30B) der Bohranordnung (10);
Aufbringen einer Last auf das Schneidwerkzeug (30B) durch Drücken des Schneidwerkzeugs (30B) gegen das Objekt (520), um das Schneidwerkzeug (30B) entlang einer Längsachse (A) der Bohranordnung (10) in eine erste Richtung verschiebbar zu bewegen und um eine druckbelastete Antriebssteueranordnung von einer inaktiven Konfiguration in eine aktive Konfiguration zu bewegen, in der die druckbelastete Antriebssteueranordnung (50B) Energie von dem Motor (512) auf das Schneidwerkzeug (30B) überträgt, um das Schneidwerkzeug (30B) anzutreiben; und
Schneiden durch das Objekt (520), derart, dass das Schneidwerkzeug (30B) von der Last entlastet wird, wobei dadurch dem Vorspannelement (96B) ermöglicht wird, das Schneidwerkzeug (30B) entlang der Längsachse (A) in eine zweite Richtung zu bewegen, die der ersten Richtung entgegengesetzt ist, und die druckbelastete Antriebssteueranordnung (50B) in die inaktive Konfiguration bewegt, um das Schneidwerkzeug (30B) nicht anzutreiben.

8. Verfahren nach Anspruch 7, wobei in der aktiven Konfiguration ein Antriebselement (54B) mit einem angetriebenen Element (52B) der druckbelasteten Antriebssteueranordnung (50B) zusammenwirkt.

9. Verfahren nach Anspruch 7 oder 8, wobei in der inaktiven Konfiguration das Antriebselement (54B) von dem angetriebenen Element (52B) der druckbelasteten Antriebssteueranordnung (50B) beabstandet ist.

10. Verfahren nach einem der Ansprüche 7 bis 9, ferner umfassend Durchschneiden des Objekts (520) mit einer von einer Fräse, einem Bohreinsatz oder einer Säge.

## Revendications

1. Ensemble de forage (10) comprenant :
un outil de coupe (30B) mobile de manière coulissante le long d'un axe longitudinal (A) de l'ensemble de forage (10) et pouvant être entraîné pour couper un objet (520) ;
un ensemble de commande d'entraînement sous pression (50B) configurable dans une configuration active et une configuration inactive, dans la configuration active, l'ensemble de commande d'entraînement sous pression (50B) transfère l'énergie d'un moteur (512) à l'outil de coupe (30B) pour entraîner l'outil de coupe (30B), dans la configuration inactive, l'ensemble de commande d'entraînement sous pression (50B) empêche le transfert d'énergie du moteur (512) à l'outil de coupe (30B) ; et
un élément de sollicitation (96B) de l'ensemble de commande d'entraînement sous pression (50) conçu pour solliciter l'ensemble de commande d'entraînement sous pression (50) dans la configuration inactive ;
dans lequel l'appui de l'outil de coupe (30B) contre l'objet (520) applique une charge à l'outil de coupe (30B) pour déplacer l'outil de coupe (30B) le long de l'axe longitudinal (A) dans une première direction et fait passer l'ensemble de commande d'entraînement sous pression (50B) de la configuration inactive à la configuration active ;
dans lequel l'élément de sollicitation (96B) déplace l'outil de coupe (30B) le long de l'axe longitudinal (A) dans une seconde direction opposée à la première direction et ramène l'ensemble de commande d'entraînement sous pression (50B) de la configuration active à la configuration inactive lorsque la charge n'est plus appliquée à l'outil de coupe (30B) ; et
**caractérisé en ce que** l'ensemble de commande d'entraînement sous pression (50B) est une configuration d'embrayage à friction.

2. Ensemble de forage (10) selon la revendication 1, dans lequel l'outil de coupe (30B) est conçu pour couper l'os.

3. Ensemble de forage (10) selon la revendication 1 ou 2, dans lequel l'outil de coupe (30B) est conçu pour couper un objet non anatomique.

4. Ensemble de forage (10) selon l'une des revendications 1 à 3, dans lequel l'outil de coupe (30B) est l'un parmi une fraise, un trépan ou une scie.

5. Ensemble de forage (10) selon l'une des revendications 1 à 4, dans lequel l'ensemble de commande d'entraînement sous pression (50B) comporte un élément entraîné (52B) mobile en coopération avec l'outil de coupe (30B) pour entraîner l'outil de coupe (30B), l'élément entraîné (52B) se déplace à l'unisson avec l'outil de coupe (30B) le long de l'axe longitudinal (A) de l'ensemble de forage (10).

6. Ensemble de forage (10) selon l'une des revendications 1 à 5, dans lequel l'élément de sollicitation (96B) comporte un ressort.

7. Procédé de découpe d'un objet non anatomique (520), le procédé comprenant :
la liaison d'un ensemble de forage (10) selon l'une quelconque des revendications 1 à 6 à un moteur (512) pour entraîner un outil de coupe (30B) de l'ensemble de forage (10) ;
appliquer une charge à l'outil de coupe (30B) en pressant l'outil de coupe (30B) contre l'objet (520) pour déplacer de manière coulissante l'outil de coupe (30B) le long d'un axe longitudinal (A) de l'ensemble de forage (10) dans une première direction, et pour déplacer un ensemble de commande d'entraînement sous pression d'une configuration inactive à une configuration active dans laquelle l'ensemble de commande d'entraînement sous pression (50B) transfère l'énergie du moteur (512) à l'outil de coupe (30B) pour entraîner l'outil de coupe (30B) ; et
couper à travers l'objet (520) de telle sorte que l'outil de coupe (30B) soit libéré de la charge, ce qui permet à l'élément de sollicitation (96B) de déplacer l'outil de coupe (30B) le long de l'axe longitudinal (A) dans une seconde direction qui est opposée à la première direction, et déplacer l'ensemble de commande d'entraînement sous pression (50B) vers la configuration inactive pour ne pas entraîner l'outil de coupe (30B).

8. Procédé selon la revendication 7, dans lequel, dans la configuration active, un élément d'entraînement (54B) coopère avec un élément entraîné (52B) de l'ensemble de commande d'entraînement sous pression (50B).

9. Procédé selon la revendication 7 ou 8, dans lequel, dans la configuration inactive, l'élément d'entraînement (54B) est espacé de l'élément entraîné (52B) de l'ensemble de commande d'entraînement sous pression (50B).

10. Procédé selon l'une des revendications 7 à 9, comprenant en outre le découpage à travers l'objet (520) avec l'un parmi une fraise, un foret ou une scie.
